# EUROPEAN PATENT SPECIFICATION

(11) **EP 0 588 126 B1**
(45) Date of publication and mention of the grant of the patent: **29.12.1997**
(21) Application number: 93113742.6
(22) Date of filing: 27.08.1993
(51) Int. Cl.: A61N 1/39, A61N 1/365

(54) **Implantable heart defibrillator**
Implantierbares Herz-Defibrillationsgerät
Defibrillateur cardiaque implantable

(30) Priority: 16.09.1992 SE 9202663
(43) Date of publication of application: 23.03.1994
(73) Proprietor: Pacesetter AB, 171 95 Solna (SE)
(72) Inventor: Hedberg, Sven-Erik, S-196 32 Kungsängen (SE)
(74) Representative: Lettström, Richard Wilhelm

(56) References cited:
- US-A- 4 637 397
- US-A- 4 693 253
- US-A- 4 821 723
- US-A- 4 850 357
- US-A- 4 998 531
- US-A- 5 107 834

## Description

The present invention relates to an implantable heart defibrillator with at least one intracardiac defibrillation electrode.

An apparatus for detecting and treating heart arrhythmias with both pacemaker stimulation and defibrillation shocks is previously known through US-A-4 940,054 and Paul J. Troup, "Implantable Cardioverters and Defibrillators, Current Problems in Cardiology", Year Book Medical Publishers, Inc., Chicago, volume XIV, no. 12, December 1989, p. 699 ff.

Heart cells are affected by the defibrillation shock after a defibrillation pulse, making cells around the stimulation electrode hard to excite with ordinary stimulation pulses from a pacemaker.

An apparatus for providing high energy stimulation pulses after defibrillation is known from US-A-4 693 253. This apparatus uses a voltage regulator to charge a separate pacing storage capacitor to a desired voltage.

The object of the present invention is to solve this problem and produce an implantable heart defibrillator which makes possible more efficient stimulation of the heart immediately after a defibrillation pulse has been emitted.

This is achieved with a heart defibrillator of the above-described type with the features specified in claim 1.

In the defibrillator according to the invention, a current regulator is connected in series with the defibrillator capacitor for limiting the current output when stimulation pulses with increased energy are emitted. A device, such as a switch transistor whose output varies with the voltage measured across a series resistor, can be used as a current regulating component. The voltage across the defibrillator capacitor can then be set to a relatively high level, i.e. many times higher than the amplitude of the stimulation pulses with increased energy, the capacitor then containing a large amount of energy permitting the extraction of a plurality of stimulation pulses.

Thus, the microprocessor can monitor the voltage of the capacitors and connect the net capacitor when the voltage of the preceding capacitor drops too much. The entire charging operation and capacitor selection process can be pre-programmed.

Thus, one or more heart stimulation pulses with greatly increased energy in relation to the energy in a conventional pacemaker pulse are emitted by the heart defibrillator according to the invention. A larger number of heart cells are accessed in this way. A defibrillation electrode can be employed as the stimulation electrode. The defibrillator case or another defibrillation electrode can serve as the indifferent electrode.

According to advantageous embodiments of the defibrillator according to the invention, a post-therapy apparatus is arranged to emit a series of stimulation pulses with increased energy for a selectable period of time after a defibrillation pulse. The period elapsing between emission of a defibrillation pulse and emission of the first stimulation pulse and the frequency of stimulation pulses are also selectable.

The stimulation pulses with increased energy can be monophasic or biphasic, e.g. biphasic stimulation pulses.

According to other advantageous embodiments of the defibrillator according to the invention, the post-therapy apparatus contains at least one capacitor which is charged to a lower voltage than the defibrillator capacitor for emission of the stimulation pulses with increased energy. Alternately, the defibrillator according to the invention can be devised so the defibrillation capacitor is utilized for emitting the stimulation pulses with increased energy. Here, a circuit is provided for sensing the voltage across the defibrillator capacitor following a defibrillation pulse and, when necessary, limiting the charging to a lower voltage than in heart defibrillation.

The post-therapy apparatus also contains devices for shortening the duration of the stimulation pulses with increased energy compared to the duration of the defibrillation pulses.

Since the defibrillator according to the invention can advantageously even contain a pacemaker unit, a microprocessor can control both the pacemaker and defibrillation functions in the defibrillator according to the invention, both the capacitor charging and the rate of stimulation then being controlled by the microprocessor. So in this instance, the post-stimulation pulses with increased energy for follow-up treatment are sent to the defibrillation unit instead of to the pacemaker unit. After stimulation with pulses with increased energy, normal pacemaker operation with stimulation through conventional pacemaker electrodes can resume.

If a limited number of stimulation pulses with increased energy is to be emitted, the pulses can, according to another advantageous embodiment of the defibrillator according to the invention, be supplied by one and the same capacitor if the pulse durations are shortened. If a long series of stimulation pulses with increased energy is desired, the charging circuit for charging the capacitor can also be enabled during the follow-up treatment. After emission of a defibrillation pulse, the defibrillator capacitor may still hold enough energy to supply the requisite stimulation pulses with increased energy. So a circuit is provided for such instances to sense whether the defibrillator capacitor holds sufficient energy. In other instances, another capacitor, charged to an appropriate level and, according to another advantageous embodiment of the defibrillator according to the invention, the microprocessor decides, according to a program, how different capacitors should supply stimulation pulses with increased energy.

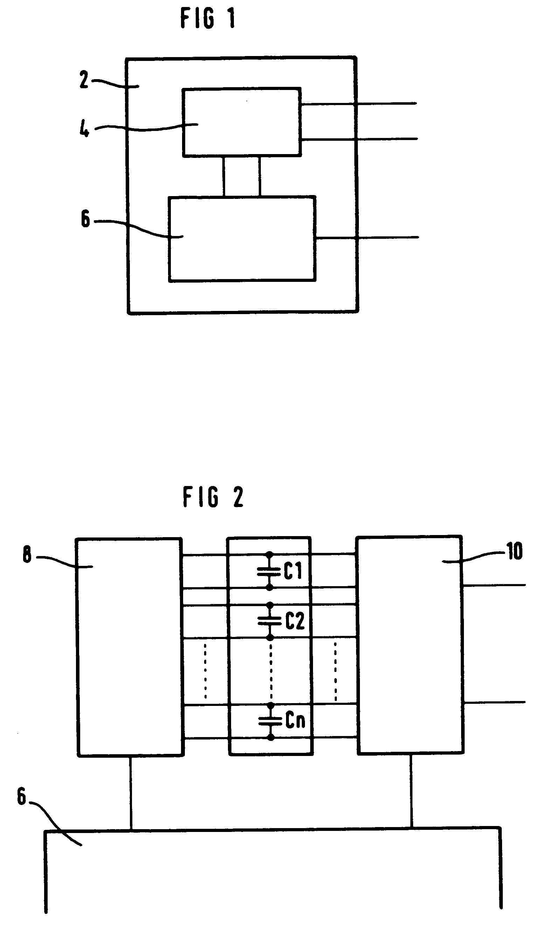

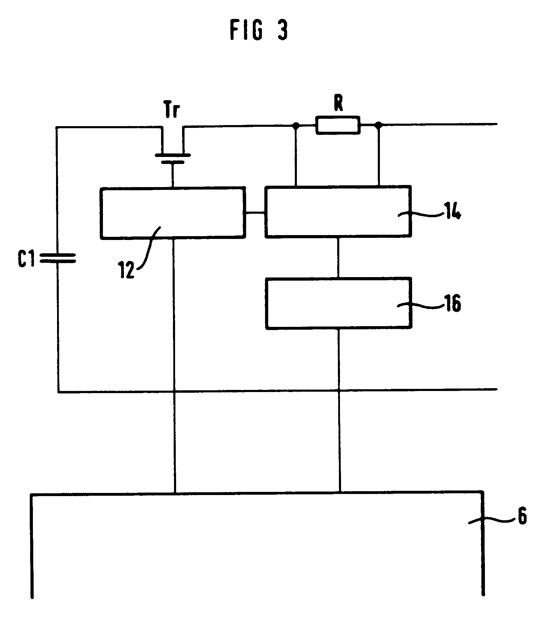

An exemplified embodiment of the defibrillation according to the invention will now be described in greater detail, referring to the accompanying drawings in which, FIG. 1 shows, in a simplified block diagram, one embodiment of the defibrillator according to the invention, FIG. 2 shows a part of the defibrillator in FIG. 1 in a more detailed block diagram and FIG. 3 shows the regulation of the output current from the capacitor when stimulation pulses with increased energy are emitted.

In FIG. 1, one embodiment of the defibrillator according to the invention is shown in a simplified block diagram. The defibrillator 2 contains an output stage 4 and other defibrillation circuits 6.

The output stage 4 contains the energy-storing capacitors, charging circuits, devices for measuring the current to the defibrillation electrodes and capacitor voltages, plus switches for controlling both defibrillation shocks and stimulation pulses with increased energy.

The unit 6 contains other parts of a defibrillator, a.o. a microprocessor for controlling the defibrillator 2. Thus, the microprocessor in the unit 6 determines which capacitor(s) in the output stage 4 are to be utilized for issuing stimulation pulses. When the energy in a capacitor becomes too low, the next capacitor is connected etc. Since the course of post-therapy stimulation is known in advance, the microprocessor can be programmed to calculate which capacitors should be used. Here, the starting point can be a probable load between the defibrillation electrodes, or the resistor can be calculated from the impedance measured between the electrodes. Both the charging procedure and the choice of capacitors can be pre-programmed. The microprocessor in unit 6 monitors the voltage across the capacitors in the output stage 4 and connects the next capacitor when the preceding capacitor's voltage falls too much. The times for the pulse emission and the pulse widths are also controlled by the microprocessor in the unit 6.

The output stage 4 contains charging circuits 8 for charging the capacitors C1, C2, ... Cn to voltages determined by the defibrillator's unit 6 (cf. FIG. 2). It is assumed that the capacitor C1 in the output stage (cf. FIG. 2) constitutes the defibrillator capacitor and that other capacitors C2, ... Cn constitute other capacitors, especially arranged to issue stimulation pulses with increased energy.

The capacitors C1, C2, ... Cn are discharged, via the switching and control unit 10, through the defibrillation electrodes. Switches in the switching and control unit 10 are controlled by the unit 6, so the desired stimulation pulses with increased energy are obtained by discharge of the capacitors C1, C2, ... Cn.

In Fig. 3, the control of the discharge of the capacitors is illustrated in greater detail.

The selection and the discharge of the capacitors appropriately take place according to a pre-programmed procedure.

Selection of capacitor C1, C2, ... Cn is made via a time control signal to the bias block 12 which then opens the transistor Tr, causing a current to flow through the resistor R. The voltage across the resistor R is measured with the measurement unit 14 and compared in the comparator 16 to a reference value specified by the microprocessor in the unit 6. When the voltage reaches the reference value, the transistor is cut off through the bias block 12 just enough to pass a current equal to the reference value. In this manner, output pulses are obtained with the desired intensity.

An additional transistor can also be connected to the capacitors. The object here is to cut any internal connection between the other capacitors and the defibrillation electrodes. However, this is previously known and will not be described here, see US-A-4, 800,883.

## Claims

1. An implantable heart defibrillator with at least one intracardiac defibrillation electrode, comprising a post-therapy apparatus (8,10,C1,C2,...Cn) arranged to emit through the defibrillation electrode after a defibrillation pulse, at least one stimulation pulse with increased energy suitable for exciting heart cells after a defibrillation pulse, in which the stimulation pulses with increased energy are emitted through discharge of the defibrillator capacitor (C1), **characterized in** that a current regulator (TR,R,12,14,16) is connected in series with the defibrillator capacitor (C1) for limiting the current output in the emission of stimulation pulses with increased energy.

2. The defibrillator of Claim 1, **characterized in** that the post-therapy apparatus (8,10,C1,C2,...Cn) is arranged to emit a series of stimulation pulses with increased energy for a selectable period of time after a defibrillation pulse.

3. The defibrillator of Claim 1 or 2, **characterized in** that the post-therapy apparatus (8,10,C1,C2,...Cn) is arranged to emit the first stimulation pulse with increased energy for a selectable period of time after a defibrillation pulse.

4. The defibrillator of Claim 2 or 3, **characterized in** that the post-therapy apparatus (8,10,C1,C2,...Cn) is arranged to emit stimulation pulses with increased energy at a selectable frequency.

5. The defibrillator of any of Claims 1-4, **characterized in** that the post-therapy apparatus (8,10,C1,C2,...Cn) contains at least one capacitor (C1,C2,...Cn) which is arranged to be charged to a lower voltage than the defibrillator capacitor (C1) for emission of the stimulation pulses with increased energy.

6. The defibrillator of any of Claims 1-5, **characterized in** that the post-therapy apparatus (8,10,C1,C2,...Cn) contains a circuit (8) for sensing the voltage across the defibrillator capacitor (C1) following a defibrillation pulse and, when necessary, limiting charging of the defibrillator capacitor (C1) to a lower voltage than in heart defibrillation for emission of stimulation pulses with increased energy, but lower energy than a defibrillation pulse, through discharge of the defibrillator capacitor.

7. The defibrillator of any of Claims 1-6, **characterized in** that the post-therapy apparatus (8,10,C1,C2,...Cn) contains devices (10) for shortening the duration of stimulation pulses with increased energy compared to the duration of the defibrillation pulses.

8. The defibrillator of any of Claims 1-7, **characterized in** that the post-therapy apparatus (8,10,C1,C2,...Cn) is arranged to discharge one and the same capacitor (C1,C2,...Cn) in a plurality of stimulation pulses with increased energy.

9. The defibrillator of any of Claims 1-8, **characterized in** that the current regulator contains a transistor (Tr) with a series resistor (R), said transistor (Tr) being controlled depending on a voltage measured across the series resistor (R).

10. The defibrillator of any of Claims 1-9, **characterized in** that a measurement unit (14) is arranged to measure the voltage across the series resistor (R), said voltage being compared in a comparator (16) with a predesignated reference voltage, and in that a bias block (12) receives the measurement signal from the measurement unit (14) and controls the transistor (Tr) to conduct a current corresponding to this reference voltage.

11. The defibrillator of any of Claims 1-9, which contains a microprocessor and in which a plurality of capacitors (C1, C2,...Cn) are dischargeable for emission of stimulation pulses with increased energy, **characterized in** that the microprocessor senses the voltages on the capacitors (C1,C2,...Cn) and decides, according to a program, how the stimulation pulses with increased energy should be extracted from the capacitors (C1,C2,...Cn).

## Patentansprüche

1. Ein implantierbarer Defibrillator mit mindestens einer intrakardialen Defibrillationselektrode, mit einem Posttherapiegerät (8, 10, C1, C2,... Cn) ausgebildet, um über die Defibrillationselektrode nach einem Defibrillationsimpuls mindestens einen Stimulationsimpuls mit erhöhter Energie, passend zur Anregung von Herzzellen nach einem Defibrillationsimpuls, abzugeben, wobei die Stimulationsimpulse mit erhöhter Energie durch Entladen des Defibrillatorkondensators (C1) abgegeben werden, **dadurch gekennzeichnet**, daß ein Stromregulator (TR, R, 12, 14, 16) in Reihe mit dem Defibrillatorkondensator (C1) geschaltet ist, um den Ausgangsstrom bei der Abgabe von Stimulationsimpulsen mit erhöhter Energie zu begrenzen.

2. Der Defibrillator nach Anspruch 1, **dadurch gekennzeichnet**, daß das Posttherapiegerät (8, 10,C1, C2,... Cn) so ausgebildet ist, daß es eine Reihe von Stimulationsimpulsen mit erhöhter Energie für eine wählbare Zeitdauer nach dem Defibrillationsimpuls abgibt.

3. Der Defibrillator nach Anspruch 1 oder 2, **dadurch gekennzeichnet**, daß das Posttherapiegerät (8, 10, C1, C2,... Cn) so ausgebildet ist, daß es den ersten Stimulationsimpuls mit erhöhter Energie für eine wählbare Zeitdauer nach dem Defibrillationsimpuls abgibt.

4. Der Defibrillator nach Anspruch 2 oder 3**, dadurch gekennzeichnet**, daß das Posttherapiegerät (8, 10, C1, C2,... Cn) so ausgebildet ist, daß es Stimulationsimpulse mit erhöhter Energie bei einer wählbaren Frequenz abgibt.

5. Der Defibrillator nach einem der Ansprüche 1-4, **dadurch gekennzeichnet**, daß das Posttherapiegerät (8, 10, C1, C2,... Cn) mindestens einem Kondensator (C1, C2,...Cn) enthält, der vorgesehen ist, um auf eine niedrigere Spannung zur Abgabe von Stimulationsimpulsen mit erhöhter Energie als der Defibrillatorkondensator (C1) aufgeladen zu werden.

6. Der Defibrillator nach einem der Ansprüche 1-5, **dadurch gekennzeichnet**, daß das Posttherapiegerät (8, 10, C1, C2,... Cn) eine Schaltung (8) zum Abfühlen der Spannung über dem Defibrillatorkondensator (C1) nach einem Defibrillationsimpuls enthält und, wenn nötig, zum Begrenzen des Aufladens des Defibrillatorkondensators (C1) auf eine niedrigere Spannung als bei Herzdefibrillation zur Abgabe von Stimulationsimpulsen mit erhöhter, aber niedrigerer Energie als ein Defibrillationsimpuls durch Entladen des Defibrillatorkondensators.

7. Der Defibrillator nach einem der Ansprüche 1-6, **dadurch gekennzeichnet**, daß das Posttherapiegerät (8, 10, C1, C2,... Cn) Vorrichtungen (10) zum Verkürzen der Dauer von Stimulationsimpulsen mit erhöhter Energie verglichen zu der Dauer des Defibrillationsimpulses enthält.

8. Der Defibrillator nach einem der Ansprüche 1-7, **dadurch gekennzeichnet**, daß das Posttherapiegerät (8, 10, C1, C2, ...Cn) so ausgebildet ist, daß es ein und denselben Kondensator (C1, C2, ...Cn) in einer Vielzahl von Stimulationsimpulsen mit erhöhter Energie entläd.

9. Der Defibrillator nach einem der Ansprüche 1-8, **dadurch gekennzeichnet**, daß der Stromregulator einen Transistor (Tr) mit einem Reihenwiderstand ( R ) enthält, wobei der Transistor (Tr) in Abhängigkeit von einer über dem Reihenwiderstand gemessenen Spannung gesteuert wird.

10. Der Defibrillator nach einem der Ansprüche 1-9, **dadurch gekennzeichnet**, daß eine Meßeinheit (14) vorgesehen ist, um die Spannung über dem Reihenwiderstand (R) zu messen, daß diese Spannung in einem Komparator (16) mit einer vorbestimmten Referenzspannung verglichen wird und daß ein Vorspannungsblock (12) das Meßsignal von der Meßeinheit (14) erhält und den Transistor (Tr) steuert, um einen zu dieser Referenzspannung korrespondierenden Strom zu leiten.

11. Der Defibrillator nach einem der Ansprüche 1-9, der einen Mikroprozessor enthält und in dem eine Mehrzahl von Kondensatoren (C1, C2,... Cn) zur Abgabe von Stimulationsimpulsen mit erhöhter Energie entladbar sind, **dadurch gekennzeichnet**, daß der Mikroprozessor die Spannung an den Kondensatoren (C1, C2, ...Cn) abfühlt und gemäß einem Programm entscheidet, wie die Stimulationsimpulse mit erhöhter Energie von den Kondensatoren (C1, C2, ...Cn) gewonnen werden sollten.

## Revendications

1. Défibrillateur cardiaque implantable comportant au moins une électrode de défibrillation intracardiaque, comportant un dispositif (8, 10, C1, C2,...Cn) post-thérapeutique agencé pour émettre par l'électrode de défibrillation après une impulsion de défibrillation, au moins une impulsion de stimulation ayant une énergie accrue appropriée pour exciter des cellules cardiaques après une impulsion de défibrillation, dans lequel les impulsions de stimulation ayant une énergie accrue sont émises par la décharge du condensateur (C1) du défibrillateur, caractérisé en ce que un dispositif (TR, R, 12, 14, 16) de régulation de courant est relié en série au condensateur (C1) de défibrillateur pour imiter la sortie de courant dans l'émission des impulsions de stimulation ayant une énergie accrue.

2. Défibrillateur suivant la revendication 1, caractérisé en ce le dispositif (8, 10, C1, C2...Cn) post-thérapeutique est agencé pour émettre une série d'impulsions de stimulation ayant une énergie accrue pendant une période de temps pouvant être choisie après une impulsion de défibrillation.

3. Défibrillateur suivant la revendication 1 ou 2, caractérisé en ce que le dispositif (8, 10, C1, C2...Cn) post-thérapeutique est agencé pour émettre la première impulsion de stimulation ayant une énergie accrue pour une période de temps pouvant être choisie après une impulsion de défibrillation.

4. Défibrillateur suivant la revendication 2 ou 3, caractérisé en ce que le dispositif (8, 10, C1, C2...Cn) post-thérapeutique est agencé pour émettre des impulsions de stimulation ayant une énergie accrue à une fréquence pouvant être choisie.

5. Défibrillateur suivant l'une quelconque des revendications 1 à 4, caractérisé en ce que le dispositif (8, 10, C1, C2...Cn) post-thérapeutique comporte au moins un condensateur (C1, C2, ...Cn) qui est agencé pour être chargé à une tension inférieure à celle du condensateur (C1) du défibrillateur destiné à l'émission d'impulsions de stimulation ayant une énergie accrue.

6. Défibrillateur suivant l'une quelconque des revendications 1 à 5, caractérisé en ce que le dispositif (8, 10, C1, C2...Cn) post-thérapeutique contient un circuit (8) destiné à détecter la tension aux bornes du condensateur (C1) de défibrillateur à la suite d'une impulsion de défibrillation et, lorsque cela est nécessaire, à limiter la charge du condensateur (C1) de défibrillateur à une tension inférieure à celle dans une défibrillation cardiaque pour l'émission d'impulsions de stimulation ayant une énergie accrue, mais une énergie inférieure à une impulsion de défibrillation, par la décharge du condensateur de défibrillateur.

7. Défibrillateur suivant l'une quelconque des revendications 1 à 6, caractérisé en ce que le dispositif (8, 10, C1, C2,...Cn) post-thérapeutique comporte des dispositifs (10) destinés à raccourcir la durée des impulsions de stimulation ayant une énergie accrue par comparaison à la durée des impulsions de défibrillation.

8. Défibrillateur suivant l'une quelconque des revendications 1 à 7, caractérisé en ce que le dispositif (8, 10, C1, C2,...Cn) post-thérapeutique est agencé pour décharger un unique et même condensateur (C1, C2,...Cn) en une pluralité d'impulsions de stimulation ayant une énergie accrue.

9. Défibrillateur suivant l'une quelconque des revendications 1 à 8, caractérisé en ce que le dispositif de régulation de courant comporte un transistor (Tr) ayant une résistance (R) en série, le transistor (Tr) étant commandé en fonction d'une tension mesurée aux bornes de la résistance (R) série.

10. Défibrillateur suivant l'une quelconque des revendications 1 à 9, caractérisé en ce qu'une unité (14) de mesure est agencée pour mesurer la tension aux bornes de la résistance (R) de série, la tension étant comparée dans un comparateur (16) à une tension de référence pré-désignée et en ce qu'un bloc (12) de polarisation reçoit le signal de mesure de l'unité (14) de mesure et commande le transistor (Tr) pour conduire un courant correspondant à cette tension de référence.

11. Défibrillateur suivant l'une quelconque des revendications 1 à 9, qui contient un microprocesseur et dans lequel une pluralité de condensateurs (C1, C2,...Cn) peuvent être déchargés pour l'émission d'impulsions de stimulation ayant une énergie accrue, caractérisé en ce que le microprocesseur détecte les tensions sur les condensateurs (C1, C2,...Cn) et décide, en fonction d'un programme, comment les impulsions ayant une énergie accrue doivent être extraites des condensateurs (C1, C2,...Cn).
